# EUROPEAN PATENT APPLICATION

(11) **EP 4 671 260 A2**
(43) Date of publication of application: **31.12.2025**
(21) Application number: 25185782.7
(22) Date of filing: 27.06.2025
(51) Int. Cl.: C07J 7/00, C07J 9/00, C07J 41/00

(54) **PROCESS FOR THE PREPARATION OF (3-BETA,24S)-25,25,25-TRIFLUORO-3-METHYL-26,27-DINORERGOST-5-ENE-3,24-DIOL**

(30) Priority: 28.06.2024 IT 202400014941
(71) Applicant: Industriale Chimica S.r.l., 20145 Milano (IT)
(72) Inventor: FASANA, Andrea, 22020 Nesso (IT); LENNA, Roberto, 20010 S. GIORGIO SU LEGNANO (IT); BARBIERI, Francesco, 21022 Azzate (IT); MICALI, Dario Liborio, 21047 Saronno (IT)
(74) Representative: Palladino, Saverio Massimo

(57) **Abstract**

The present invention relates to a process for the industrial scale preparation of (3β,24*S*)-25,25,25-trifluoro-3-methyl-26,27-dinorergost-5-ene-3,24-diol, useful in counteracting the progression of highly disabling neurological diseases such as Alzheimer's, Parkinson's, and Huntington's disease, and having the structure shown below:

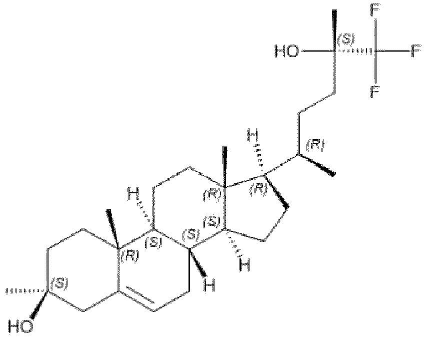

The invention also relates to an improved method for the separation of two epimers that are obtained during the synthesis.

## Description

### FIELD OF THE INVENTION

The present invention refers to the sector of processes for the synthesis of active ingredients for pharmaceutical use, and in particular to a process for the industrial scale preparation of (3β,24*S*)-25,25,25-trifluoro-3-methyl-26,27-dinorergost-5-ene-3,24-diol, having the structure shown below:

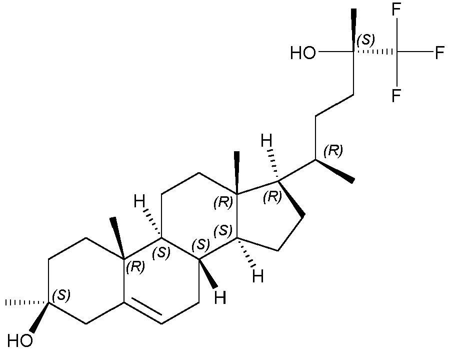

The compound is identified with the CAS Registry Number 1629853-48-0, and is also known by the use names of SAGE-718 or Dalzanemdor; in the following description the compound will be referred to as SAGE-718.

SAGE-718 belongs to the class of neuroprotective agents; these derivatives can be used in the preparation of drugs having a pharmacological activity useful for counteracting the progression of highly disabling neurological diseases such as Alzheimer's, Parkinson's, and Huntington's disease.

The molecule is currently in the clinical trial phase to evaluate the efficacy, tolerability, and safety profile thereof.

### STATE OF THE ART

The compound SAGE-718 was first described in the article "SAGE-718: A first-in-class N-methyl-D-aspartate receptor positive allosteric modulator for the potential treatment of cognitive impairment", M. D. Hill et al., J. Med. Chem. 2022, 65, 9063-9075, which for brevity will also be referred to hereinafter simply as the "article by Hill". In addition to the stereo centers of the steroid skeleton (indicated in the formula above), the compound is characterized by the presence of a stereocenter also in position 24 on the side chain.

Of the two possible epimers, the one with the S configuration in position 24 is of pharmaceutical interest.

The synthesis scheme of the article by Hill is indicated in schemes 1 and 2 on page 9065. In scheme 2, the separation step from intermediate 15 (epimeric mixture) to compounds 5 (SAGE-718) and 16 refers to the purification carried out by chromatographic resolution on a preparative column, operating on the epimeric mixture obtained from the synthesis with supercritical CO₂ on a chiral stationary phase in reverse phase.

This purification method can be used at the laboratory level when the obtainment of small amounts of pure product for identification is needed; however, this is not acceptable for industrial-scale production due to operating costs, complexity of the technique and specificity of the equipment used.

The object of the present invention is to provide a synthesis and purification process for the compound SAGE-718 that is simpler than the one described in the article by Hill and therefore easier to apply industrially.

### SUMMARY OF THE INVENTION

In a first aspect thereof, the invention relates to a synthesis process that allows to obtain the epimeric mixture 15 described in the article by Hill with an overall yield of 8% as compared to 4% of the synthesis route of said article. This process comprises the following steps:
A) introduction of a methylene group in position 20 of the compound 5-pregnenolone via Wittig reaction, obtaining intermediate 8: 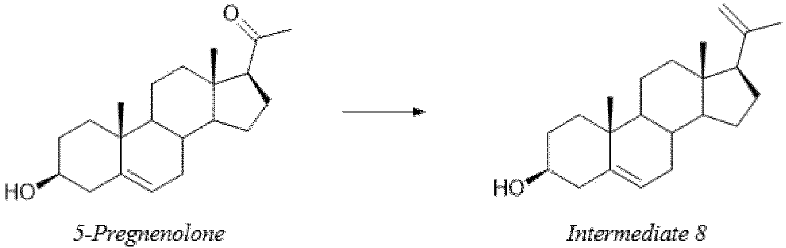
B) oxidation to carbonyl of the hydroxyl in position 3 of intermediate 8 obtaining intermediate 9: 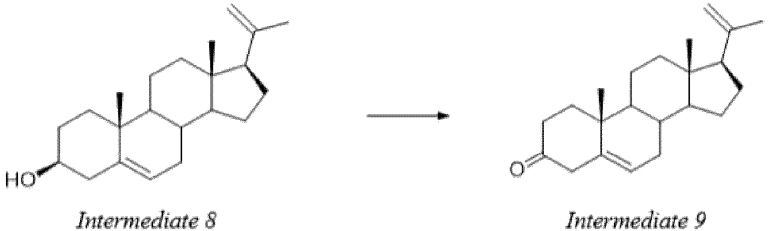
C) introduction of a methyl group in position 3 of intermediate 9 via Grignard reaction obtaining intermediate 10: 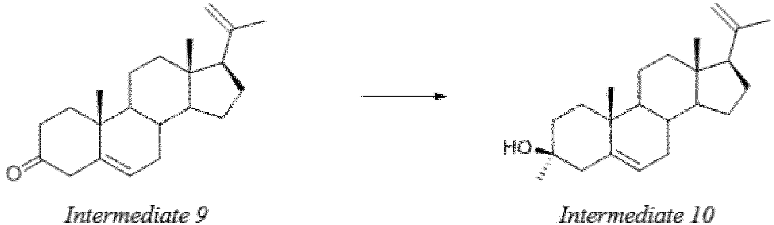
D) hydroboration-oxidation of the methylene group in position 21 of intermediate 10 obtaining intermediate 11: 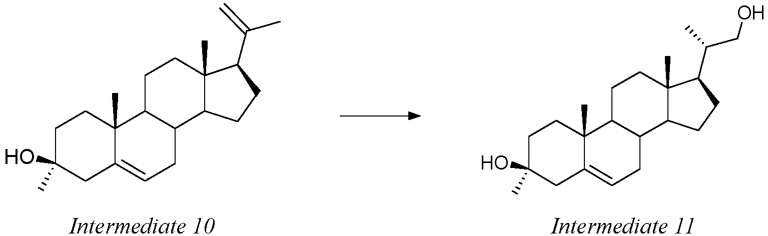
E) oxidation to carbonyl of the hydroxyl in position 22 of intermediate 11 obtaining intermediate 12: 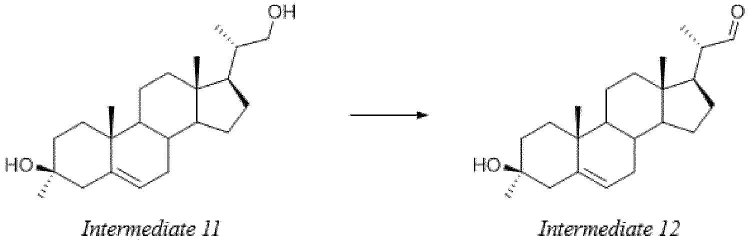
F) introduction of a 2-propanone radical in position 22 of intermediate 12 via Wittig reaction obtaining intermediate 13: 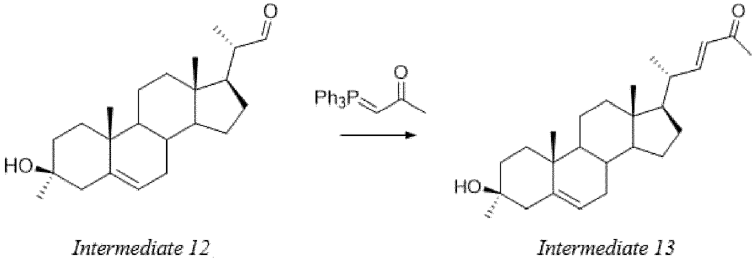
G) reduction of the double bond in position 22 of intermediate 13 obtaining intermediate 14: 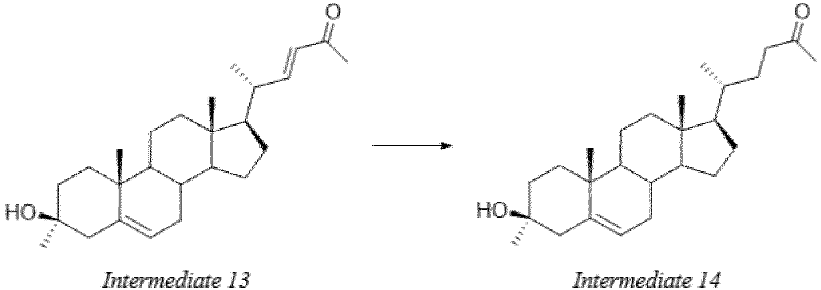
H) introduction of a trifluoromethyl radical in position 24 of intermediate 14 obtaining the epimeric mixture 15:

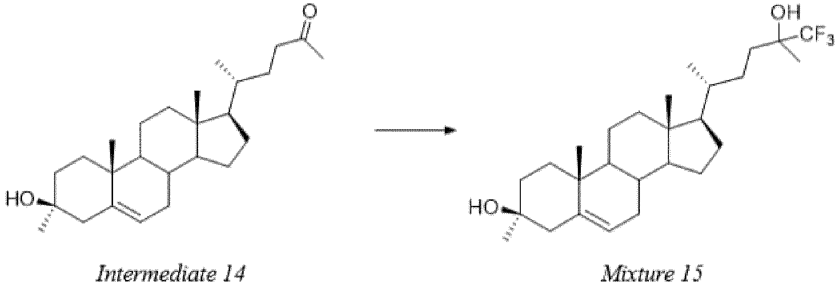

In the second aspect thereof, the invention relates to a procedure for the separation of mixture 15 to obtain the epimer S in position 24, *i.e.* the compound SAGE-718; this purification process is applicable to the epimeric mixture 15 obtained according to the steps A)-H) reported above, according to the procedure of the article by Hill, or by any other route.

The procedure according to this second aspect of the invention comprises the following steps:
I) esterification of the hydroxyl in position 3 of said epimeric mixture 15 with a protected aliphatic amino acid obtaining intermediate 16, in turn in the form of an epimeric mixture: 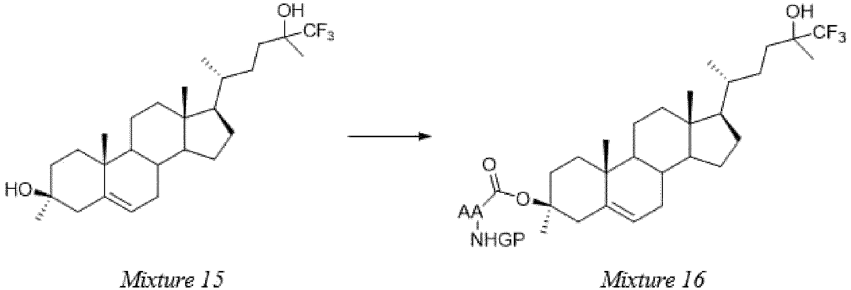 where, in the formula of mixture 16, AA indicates the radical of the amino acid and GP the protective group linked to the amino function of the amino acid;
J) chromatographic separation of the epimeric mixture 16 obtaining two epimers 17 and 18, where the epimer 17 has the desired configuration: 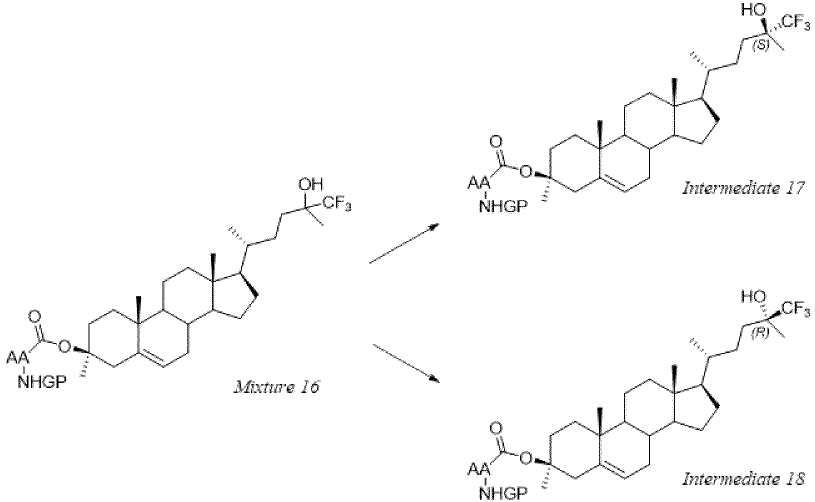
K) removal by hydrolysis of the ester in position 3 of intermediate 17, obtaining the desired product SAGE-718:

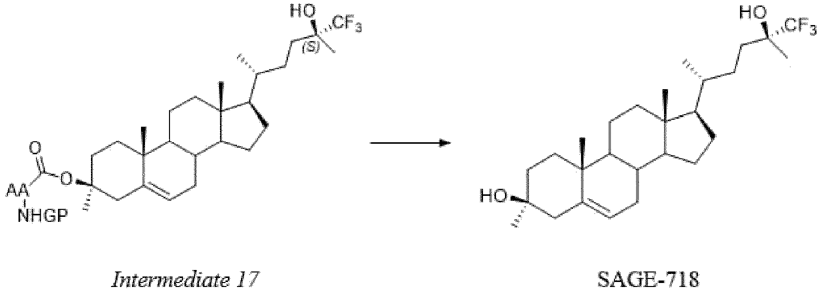

Finally, in its third aspect, the invention relates to certain intermediates of the two processes described above, namely the compound (3β)-3-hydroxy-3-methyl-26,27-dinorcholest-5,22-dien-24-one (intermediate 13) having the following formula: 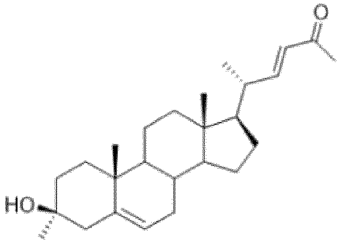
and the intermediates forming mixture 16, having the following general formula: 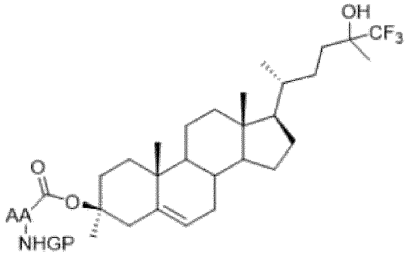
where AA represents the radical of an aliphatic amino acid and GP a protective group of the amino function of the amino acid.

### DETAILED DESCRIPTION OF THE INVENTION

With regard to the first aspect of the present invention, the starting compound is 5-pregnenolone identified with the CAS number 145-13-1, and having the formula reported below:

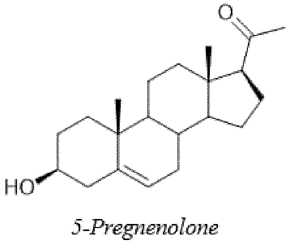

5-Pregnenolone is a steroid that has been known for years and is commonly commercially available.

The numbering of intermediates and subsequent mixtures of the synthesis route of the first aspect of the invention, from 8 to 15, was selected so as to arrive at the final product, the epimeric mixture 15, having the same numbering present in the article by Hill (as well as for intermediate 14 of the two syntheses); this in order to be able to compare the two synthesis routes more easily.

The synthesis process of the first aspect of the invention, from 5-pregnenolone to mixture 15, differs from that of the article by Hill which uses as a starting product the compound indicated in the article as compound 6; the differences in the two starting products, which are reflected in the synthesis process, are illustrated in the following figure:

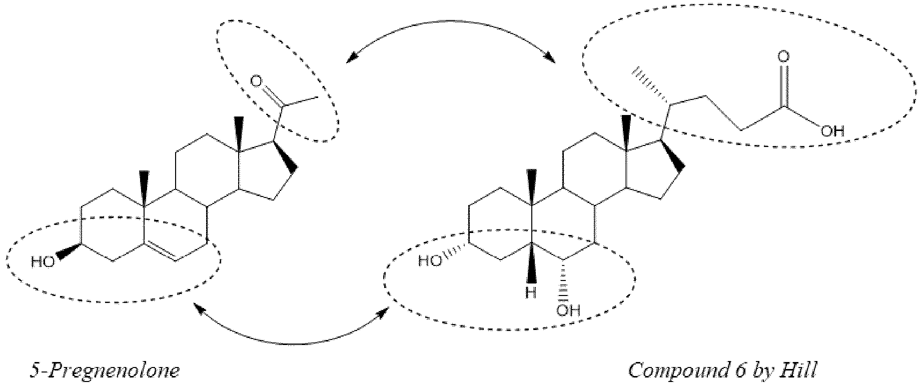

As can be observed in the starting product of the process of the invention, *i.e.* 5-pregnenolone, the double bond in position 5,6 is already present while the aliphatic chain on the fourth ring of the steroid is to be "built"; the opposite situation is found in the starting product used in the article by Hill.

Step A of the process is a Wittig reaction carried out on 5-pregnenolone in order to transform the carbonyl in position 20 into an alkene double bond (intermediate 8) that will be subsequently functionalized in step D while being inert to the reaction conditions of steps B and C.

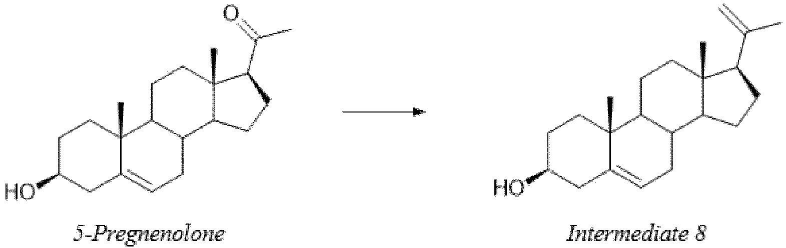

The Wittig reaction is preferably carried out in tetrahydrofuran operating at a temperature between 15 and 60 °C for a time between 1.5 and 6 hours.

The ylide is generated starting from a methyltriphenylphosphonium salt selected from methyltriphenylphosphonium bromide, methyltriphenylphosphonium chloride, methyltriphenylphosphonium iodide and methyltriphenylphosphonium methylcarbonate, used together with potassium tert-butylate; methyltriphenylphosphonium bromide is preferably used. The molar ratio of 5-pregnenolone, methyltriphenylphosphonium bromide and potassium tert-butylate is 1:1.5-2.5:1.5-2.5.

Step B is an oxidation that leads to obtainment of the carbonyl function in position 3 of the steroid skeleton:

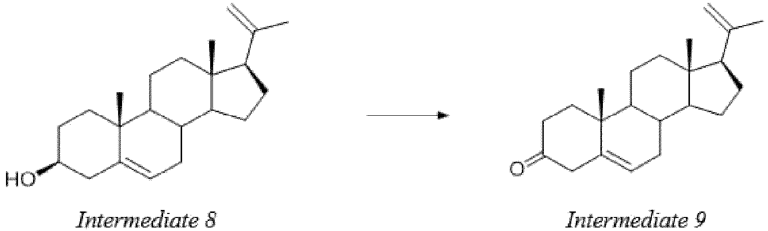

This step can be accomplished using: chromium (VI) compounds; a hypochlorite, such as calcium or sodium hypochlorite, in the presence of 2,2,6,6-tetramethylpiperidin-1-oxyl radical (known in the industry as "TEMPO") or a derivative thereof, such as 4-hydroxy-2,2,6,6-tetramethylpiperidin-1-oxyl radical, 4-methoxy-2,2,6,6-tetramethylpiperidin-1-oxyl radical, or 4-benzyloxy-2,2,6,6-tetramethylpiperidin-1-oxyl radical; ruthenium derivatives such as ruthenium tetroxide, ruthenium trichloride in the presence of a reoxidant such as sodium hypochlorite or tetrapropylammonium perruthenate in the presence of N-methylmorpholine N-oxide; potassium peroxymonosulfate KHSO₅ (commercially known as Oxone); 1,3,5-trichloro-2,4,6-triazinetrione (also known as trichloroisocyanuric acid); hypervalent iodine compounds such as Dess-Martin periodinane; or preferably, according to the present invention, using oxalyl chloride, triethylamine and dimethyl sulfoxide according to Swern conditions.

By operating according to the preferred mode (Swern conditions), the molar ratio of intermediate 8, oxalyl chloride, triethylamine and DMSO is 1:1.5-2:6-7:3-4, preferably 1:1.62:6.4:3.55.

These reaction conditions allow the obtainment of intermediate 9 in good yields by minimizing the formation of the isomerization byproduct with the double bond in the 4,5 position, the structure of which is shown below:

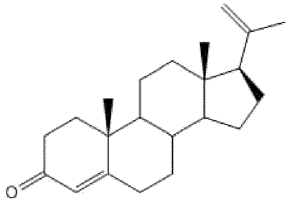

The reaction, according to this preferred mode, is carried out in dichloromethane at a temperature between -75 and 25 °C for a time between 1.5 and 6 hours, preferably between 2 and 4 hours.

The distillation of the sample solution in dichloromethane, at the end of the reaction, is carried out at reduced pressure at a temperature not exceeding 25 °C to avoid the complete conversion of intermediate 9 into the isomerization by-product with the double bond in position 4,5.

Step C leads to the insertion of a methyl group in position 3 of the steroid skeleton:

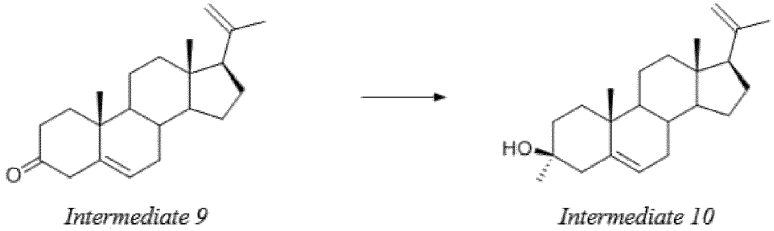

This step is stereospecific, and the methyl group selectively binds to the steroid in the α configuration. The transformation occurs by reaction of intermediate 9 with a Grignard reagent CH₃MgX, where X indicates a halogen atom selected among chlorine and bromine, in the presence of bis-(2,6-di-tert-butyl-4-methylphenoxide), generated *in situ* using trimethylaluminium and the compound 2,6-bis(1,1-dimethylethyl)-4-methylphenol, also known as butylated hydroxytoluene or by the acronym BHT. The molar ratio of intermediate 9, trimethylaluminum, BHT and the Grignard reagent is 1/3-4/6.1-8.1/3-4.

Preferably, methylmagnesium bromide (CH₃MgBr) is used in dichloromethane as a solvent at a temperature between -75 and 25 °C for a time between 3 and 6 hours.

Step D is a hydroboration-oxidation that allows the obtainment of intermediate11:

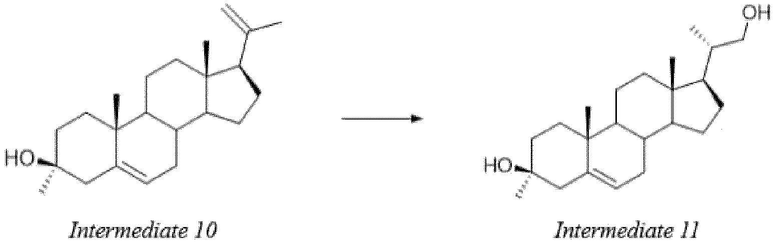

In the hydroboration step, it is possible to use the borane-tetrahydrofuran complex, the borane-diethyl ether complex, the borane-dimethylsulfide complex, the borane-pyridine complex, bis(1,2-dimethylpropyl)borane, texylborane, preferably 9-borabicyclo[3.3.1]nonane (9-BBN), and even more preferably 9-borabicyclo[3.3.1]nonane dimer (9-BBN dimer).

The reaction is preferably carried out in THF at a temperature between 5 °C and 65 °C for a time between 2 and 6 hours; preferably, it is operated at about 60 °C.

Subsequently, still as part of step D, the alkylborane intermediate formed in the previous step is oxidized with hydrogen peroxide under alkaline conditions; this oxidation introduces the -OH group in position 22 of the steroid.

Preferably, 30% by weight hydrogen peroxide and a 5M sodium or potassium hydroxide solution are used; the base is preferably sodium hydroxide.

The molar ratio of intermediate 10, 9-BBN dimer, hydrogen peroxide and sodium hydroxide is 1/1.15-3/10-15/10-15.

Step E is an oxidation of the hydroxyl introduced in the previous step:

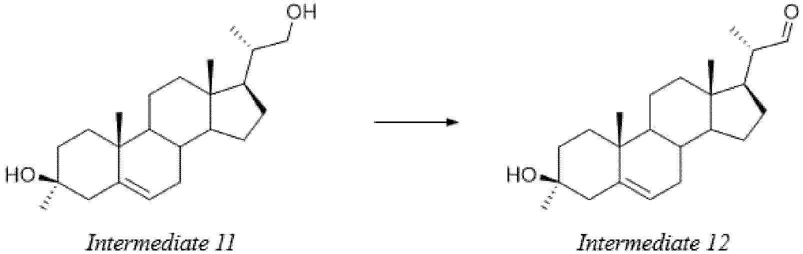

This step can be carried out using the same oxidizing systems described for step B. In this case, however, the preferred procedure is the oxidation with calcium or sodium hypochlorite in the presence of 2,2,6,6-tetramethylpiperidin-1-oxyl radical ("TEMPO"), sodium bicarbonate, and potassium or sodium bromide. When operating with these reagents, the molar ratio of intermediate 11, sodium hypochlorite, sodium bicarbonate, potassium or sodium bromide, and TEMPO is 1/1.4-2.0/0.5-1.5/0.1-0.5/0.03-0.06.

The reaction is performed in a solvent selected among dichloroethane, ethyl acetate, acetonitrile or, preferably, dichloromethane, at a temperature between 0 °C and 5 °C for a time between 2 and 4 hours. The carbonyl function thus formed enables a new Wittig reaction in the next step (F), so as to add a C3 unit to the steroid.

The Wittig reaction (step F), starting from intermediate 12, is carried out in the presence of 1-triphenylphosphoranylidene-2-propanone:

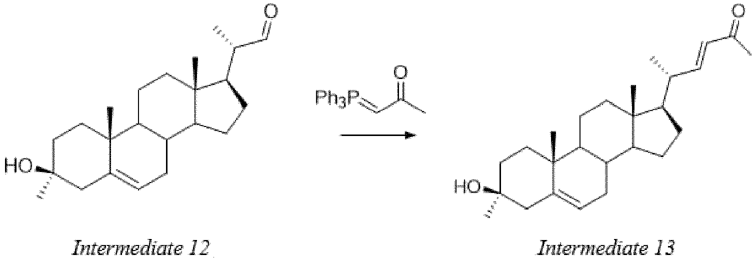

The molar ratio of intermediate 12 and 1-triphenylphosphoranylidene-2-propanone is between 1:2 and 1:4, preferably 1:3. The reaction is carried out in toluene at a temperature between 25 and 115 °C for a time between 12 and 24 hours.

Step G (from intermediate 13 to intermediate 14) is a regioselective catalytic hydrogenation on the exocyclic double bond (position 22,23) that does not reduce the other double bond in position 5,6 of the steroid:

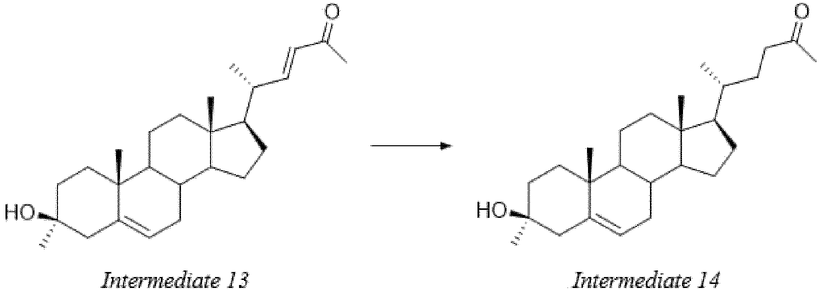

The reaction of this step consists of a hydrogenation with gaseous hydrogen in the presence of a metal catalyst dispersed on an inert substrate.

Preferred conditions for this reaction are the use of palladium on carbon (Pd/C) at 5% or 10% by weight, preferably 10% by weight, as a catalyst and the hydrogen pressure between 0.1 and 1.0 bar, preferably 0.2 bar.

Solvents that can be used to carry out this reaction are methanol, ethyl acetate and tetrahydrofuran (THF). The solvent is preferably THF. The reaction is performed at a temperature between 15 °C and 30 °C, preferably between 25 °C and for a total time of at least 16 hours.

Finally, the last step of the process according to the first aspect of the present invention, step H, consists of the transformation of intermediate 14 into the epimeric mixture 15:

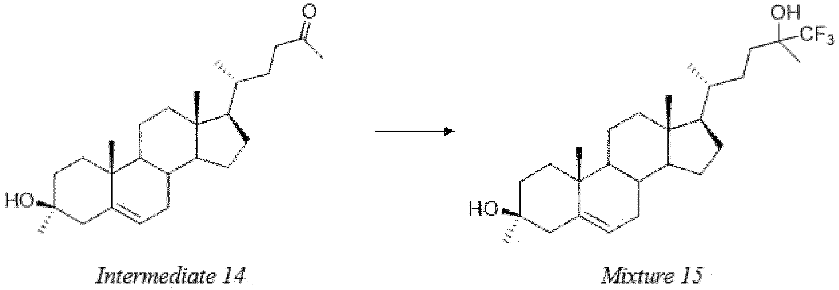

The intermediate 14, as well as the method of the transformation thereof to give the epimeric mixture 15 in step H, are the same used in the article by Hill, to which reference is made for details and operating conditions, in particular described in the example between pages 9070 and 9071 of the article, incorporated herein by reference.

In its second aspect, the invention relates to the separation of the epimeric mixture 15 to obtain the compound SAGE-718 in purified form, which is achieved through steps I to K described below. The separation process of this second aspect of the invention can be applied to the mixture regardless of the synthesis process used to obtain it and the percentage composition of the two epimers.

The purification of the invention requires a chromatographic separation like the one of the article by Hill, but unlike that, it allows to avoid the use of a chiral stationary phase and the use of CO₂ in supercritical conditions, which in turn requires the use of a pressure-tight system.

An essential element of this second aspect of the invention is step I, consisting of the selective functionalization of the hydroxyl group (-OH) in position 3 of the steroid with a protected amino acid (AANHGP) as indicated in the following scheme:

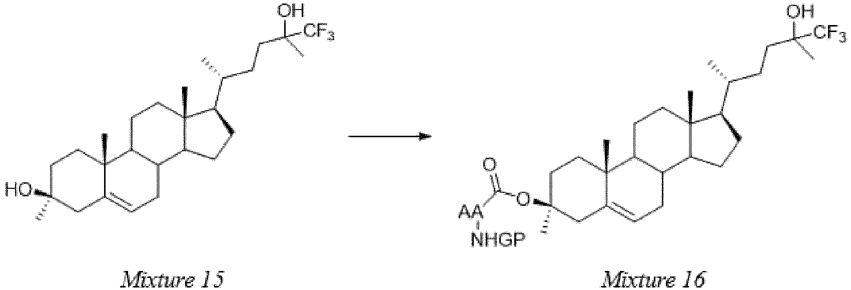

In the steroid in question there are two -OH groups (positions 3 and 24), both tertiary, but only the one in position 3 reacts. This selective functionalization allows to separate the mixture of the two epimers using a common non-chiral C8 stationary phase eluting with an acetonitrile/water mixture operating in isocratic mode.

Step I consists of the esterification of the hydroxyl in position 3 of the epimeric mixture 15 with an aliphatic amino acid:

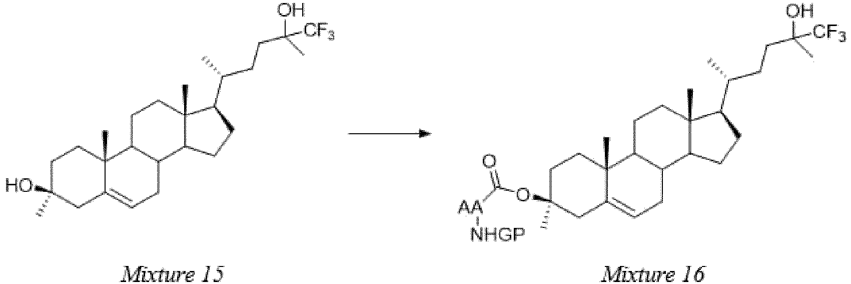

Amino acids that can be used are aliphatic ones, such as Alanine, Glycine, Valine, Leucine and Isoleucine. Preferably the amino acid is Alanine or Valine. The amino acid is used in a molar ratio between 2 and 6 with respect to mixture 15.

The amino acid protecting group (GP) is selected from carbobenzyloxy (Cbz), fluorenyl methyloxycarbonyl (Fmoc) and, preferably, tert-butyloxycarbonyl (Boc); the structures reported below show the structure of these protecting groups linked to an amino acid radical indicated as AA:

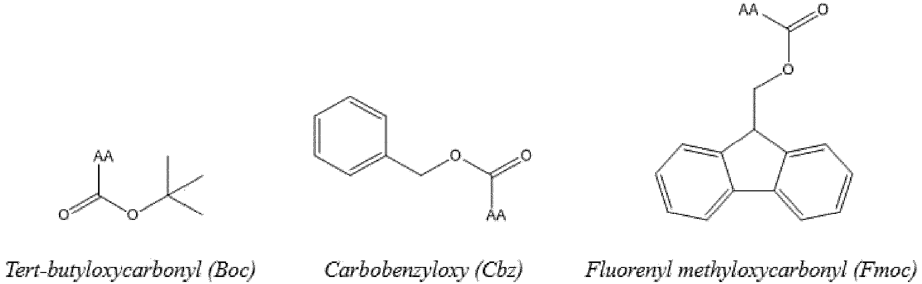

The esterification reaction of this step can be carried out using a known activating agent, such as carbodiimides, phosphonium salts such as benzotriazolyloxytris(dimethylamino)phosphonium hexafluorophosphate (BOP), 1H-benzotriazol-1-yloxytripyrrolidinophosphonium hexafluorophosphate (PyBOP) and the like, uronium salts such as 1H-benzotriazolium-1-[bis(dimethylamino)methylene]-3-oxide hexafluorophosphate (HBTU), 1-[bis(dimethylamino)methylene]-1H-1,2,3-triazolo[4,5-b]pyridinium 3-oxide hexafluorophosphate (HATU), 1-(ethoxycarbonyl)-2-ethoxy-1,2-dihydroquinoline (EEDQ), or 1-methyl-2-chloropyridinium iodide (Mukaiyama reagent).

The preferred activating agents are carbodiimides, such as 1-ethyl-3-(3-dimethylaminopropyl)carbodiimide hydrochloride (EDCI), the corresponding free base (EDC), diisopropyl carbodiimide (DIC) or dicyclohexyl carbodiimide (DCC), in the presence of catalysts such as 4-dimethylaminopyridine (DMAP) or 1-hydroxybenzotriazole (HOBt).

The preferred procedure for carrying out this step is the esterification with N,N'-dicyclohexyl carbodiimide (DCC) as the activating agent in the presence of DMAP as the catalyst. In particular, DCC is used in a molar ratio between 2 and 6 with respect to mixture 15, and DMAP is used in molar ratio amounts between 1 and 4 with respect to mixture 15.

The reaction is preferably carried out in dichloromethane at a temperature between 15 and 25 °C for a time between 2 and 18 hours.

Step J is the chromatographic separation of mixture 16:

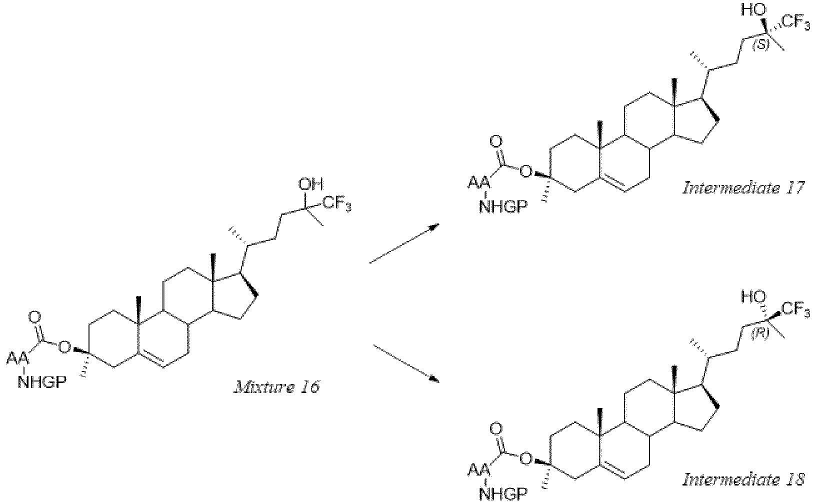

The separation of the two epimers 17 and 18 functionalized with a protected amino acid can be carried out by chromatography.

Preferably, the chromatography is carried out under the following conditions:
- Stationary phase: C4, C18 or, preferably, C8;
- Mobile phase such as: H₂O/acetonitrile/methanol or, preferably, H₂O/acetonitrile;
- Elution flow: from 10 to 40 mL/min, preferably 30 mL/min;
- Solvents to solubilize the sample: THF, ethyl acetate, isopropyl acetate or, preferably, acetonitrile;
- UV detector wavelength: 195-210 nm, preferably 200 nm.

The conditions described above allow the separation and recovery of the two epimers in pure form and are also applicable to the corresponding epimers functionalized with Boc-Glycine, Boc-Valine, Boc-Leucine and Boc-Isoleucine.

Step K, applied on intermediate 17, allows to obtain the compound SAGE-718, objective of the invention:

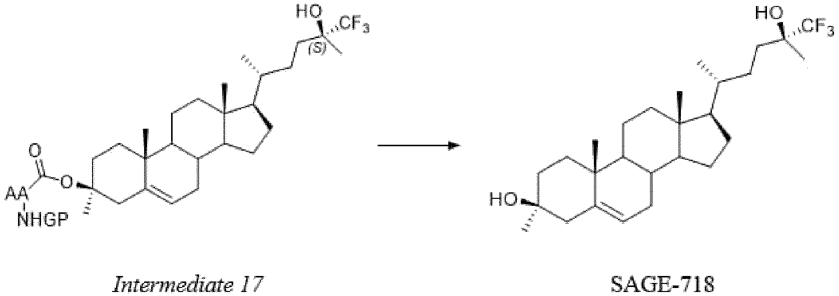

The reaction consists of the hydrolysis of the ester function of intermediate 17, through the use of bases. Preferred conditions for this reaction are:
- use of sodium carbonate, lithium carbonate, or preferably potassium carbonate as a base;
- reaction time of at least 12 hours, preferably at least 16 hours;
- solvents: acetonitrile or, preferably, THF and MeOH;
- reaction temperature between 25 and 65 °C, preferably 65 °C.

Finally, in its third aspect, the invention relates to certain intermediates of the two processes described above, namely the compound (3β)-3-hydroxy-3-methyl-26,27-dinorcholest-5,22-dien-24-one (intermediate 13) having the following formula: 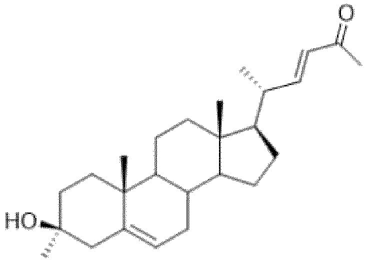
and the intermediates forming mixture 16, having the following general formula: 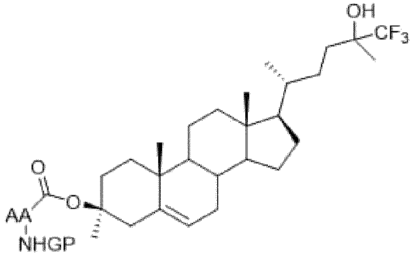
where AA represents the radical of an aliphatic amino acid and GP a protecting group of the amino function of the amino acid.

### EXPERIMENTAL PART

### INSTRUMENTS, METHODS AND EXPERIMENTAL CONDITIONS

### NMR:

NMR Spectrometer JEOL 400 YH (400 MHz); Software JEOL Delta v5.1.1;
Spectra were recorded in CDCl₃ and DMSO-d₆.

### MS:

Instrument: Agilent 1260 Infinity. Mass Spectrometer: 6120 single quadrupole

### UPLC:

Chromatographic system: Waters Acquity UPLC H-Class Plus Model

### UPLC Method:

Mobile phase A: Water + 0.01% formic acid
Mobile phase B: Acetonitrile + 0.01% formic acid
Gradient:

| Time (minutes) | Mobile Phase A | Mobile Phase B |
|---|---|---|
| 0 | 90 | 10 |
| 5 | 10 | 90 |
| 6 | 10 | 90 |
| 6.1 | 90 | 10 |
| 7 | 90 | 10 |

Chromatographic conditions:

| | |
|---|---|
| Column: | Acquity UPLC BEH C18, 2.1 x 50 mm; 1.7 µm |
| Flow rate: | 0.5 mL/minute |
| Detector: | 200 nm |
| Injection volume: | 1 µL |
| Temperature: | 35 °C |

### HPLC:

Chromatography System: Agilent 1260 Infinity model; UV Detector G1315C DAD VL+ Model

### HPLC Method for SAGE-718:

Mobile phase A: Water
Mobile phase B: Acetonitrile
Gradient:

| Time (minutes) | Mobile Phase A | Mobile Phase B |
|---|---|---|
| 0 | 70 | 30 |
| 1 | 70 | 30 |
| 20 | 20 | 80 |
| 35 | 20 | 80 |
| 35.1 | 70 | 30 |
| 45 | 70 | 30 |

Chromatographic conditions:

| | |
|---|---|
| Column: | Gemini NX-C18, 150 x 4.6 mm; 3 µm |
| Flow rate: | 0.8 mL/minute |
| Detector: | 200 nm |
| Injection volume: | 10 µL |
| Temperature: | 25 °C |

### HPLC method for products functionalized with Boc-alanine, Boc-valine, Boc-glycine:

### Chromatographic conditions:

| | |
|---|---|
| Mobile phase: | Water/Acetonitrile/MeOH 15:78.5:6.5 v/v (isocratic) |
| Column: | Robusta^{®} C8 5u - 250 mm x 4.6 mm |
| Flow rate: | 1.5 mL/minute |
| Detector: | 200 nm |
| Injection volume: | 10 µL |
| Temperature: | 25 °C |

### TLC:

MERCK: TLC silica gel 60 F₂₅₄ Aluminum sheets 20 x 20 cm, cod. 1.0554.0001.

### TLC Developer reagent:

Cerium phosphomolybdate: 25 g of phosphomolybdic acid and 10 g of cerium (IV) sulfate are dissolved in 600 mL of H₂O. 60 mL of 98% H₂SO₄ are added, and the resulting mixture is brought to 1 L with H₂O. The plate is impregnated with the solution and then heated until the products are detected.

### NOTE

The water used in the experimental descriptions is to be understood as pure water, unless otherwise indicated.

The organic solvents used in the experimental descriptions are to be understood as of "technical" grade, unless otherwise indicated.

The reagents and catalysts used in the experimental descriptions are to be understood as of commercial quality, unless otherwise indicated.

The indication "small volume", referring to a volume of solution after a distillation phase, indicates a final volume of less than half the starting volume.

The indication "reduced pressure", used in the following examples, means a pressure of less than 50 mbar.

### EXAMPLE 1

This example refers to step A) of the process of the invention.

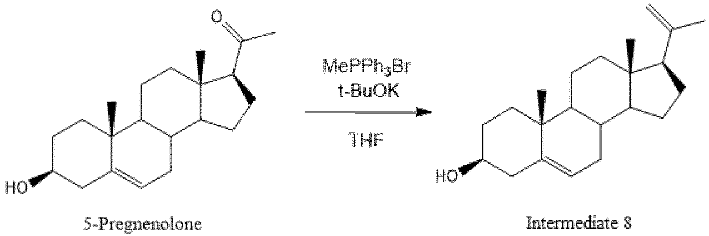

112.9 g of methyltriphenylphosphonium bromide (316.0 mmol, 2 eq) and 250 mL of tetrahydrofuran were loaded into a flask under nitrogen.

35.5 g of potassium tert-butylate (316.0 mmol, 2 eq) were added portionwise to the suspension cooled to 15 °C. The system was heated to 50 °C and stirred for 30 minutes.

50.0 g of 5-pregnenolone (158.0 mmol, 1 eq) were added portionwise to the reaction mixture, while maintaining the temperature below 60 °C.

The system was stirred for 2 hours at 50 °C.

The reaction was monitored by TLC analysis under the following conditions: TLC plate: silica gel on aluminium; starting substrate dissolved in dichloromethane; reaction mixture quenched in saturated NH₄Cl solution and extracted with ethyl acetate (EtOAc); eluent: heptane/EtOAc 1/1; developer reagent: cerium phosphomolybdate.

Once the reaction was completed, the solution was cooled to 15 °C and a 15% ammonium chloride solution (120 mL) was added dropwise.

The phases were separated, and the aqueous phase was extracted with ethyl acetate.

The organic phase was concentrated under reduced pressure to a waxy residue (137.5 g).

81 mL of methanol and 81 mL of water were added to the residue, and the system was heated to reflux for 30 minutes.

The suspension was cooled to 25 °C and stirred for 1 hour.

The solid was filtered through a Büchner filter, washing first with a 1/1 water and methanol solution, and then with water.

The solid was dried under reduced pressure at 55 °C for 6 hours.

49.1 g of product consisting of intermediate 8 (white solid) were obtained.

¹H-NMR (400 MHz, CDCl₃) δ 5.34 (m, 1H), 4.84 (s, 1H), 4.70 (s, 1H), 3.55-3.47 (m, 1H), 2.31-2.19 (m, 2H), 2.04-1.95 (m, 3H), 1.89-1.62 (m, 9H), 1.60-1.39 (m, 5H), 1.24-1.03 (m, 4H), 1.01 (s, 3H), 0.99-0.91 (m, 1H), 0.57 (s, 3H).

Mass spectrometry (ESI+): m/z = 297 [M+1-H₂O].

### EXAMPLE 2

This example refers to the implementation of step B) of the process of the invention.

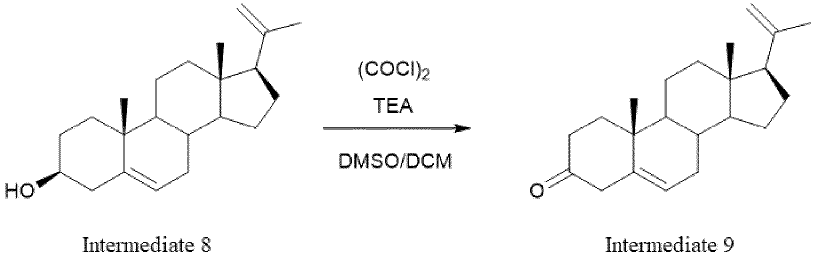

1.77 mL of oxalyl chloride (20.60 mmol, 1.62 eq) and 50 mL of methylene chloride were loaded into a flask under nitrogen.

A mixture of dimethyl sulfoxide (3.2 mL, 45.15 mmol, 3.55 eq) and methylene chloride (5 mL) was added dropwise into the solution cooled to -75 °C, while maintaining the temperature below -60 °C.

The system was brought to -70 °C and stirred for 30 minutes.

A solution of intermediate 8 (4 g, 12.72 mmol), obtained as described in the previous example, in methylene chloride (37.5 mL) was added to the reaction mixture, while maintaining the temperature below -60 °C.

The system was brought to -70 °C and stirred for 30 minutes.

11.3 mL of triethylamine (81.39 mmol, 6.4 eq) were added to the reaction mixture, while maintaining the temperature below -60 °C.

The system was stirred at -70 °C for 30 min, and then at 25 °C for 2 hours.

The reaction was monitored by TLC analysis under the following conditions: TLC plate: silica gel on aluminium; starting substrate dissolved in dichloromethane; reaction mixture quenched in water and extracted with EtOAc; eluent: heptane/EtOAc 1/1; developer reagent: cerium phosphomolybdate.

Once the reaction was completed, 32 mL of water were added to the system.

The phases were separated, and the aqueous phase was extracted with methylene chloride (32 mL).

The phases were separated, and the organic phase was washed with a sodium hypochlorite solution, with a 5% citric acid solution and then with water.

The organic phase was concentrated under reduced pressure at 25 °C to a residual volume of 14 mL.

The residue was taken up with 40 mL of isopropyl ether and reconcentrated twice to a residual volume of 14 mL.

The suspension was cooled to 0 °C and stirred for 1 hour.

The solid was filtered through a Büchner filter, washing with cold isopropyl ether.

The solid was dried under reduced pressure at 25 °C for 2 hours.

3.3 g of yellow solid consisting of intermediate 9 were obtained.

¹H-NMR (400 MHz, CDCl₃) δ 5.34 (m, 1H), 4.85 (s, 1H), 4.71 (s, 1H), 3.27 (dt, J= 16.4, 3.0 Hz, 1H), 2.82 (dd, J= 16.4, 2.1, 1H), 2.52-2.43 (m, 1H), 2.33-2.26 (m, 1H), 2.06-2.01 (m, 3H), 1.92-1.78 (m, 2H), 1.76 (s, 3H), 1.18 (s, 3H), 1.73-1.02 (m, 11H), 0.61 (s, 3H).

Mass spectrometry (ESI+): m/z = 313 [M+1].

### EXAMPLE 3

This example refers to the implementation of step C) of the process of the invention.

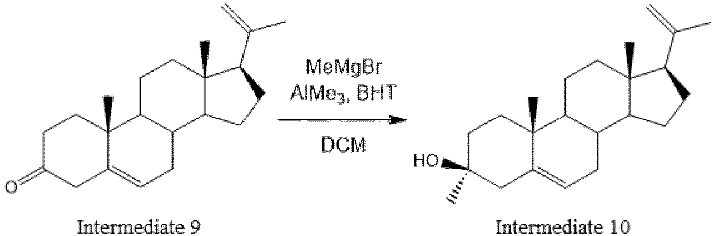

8.7 g of BHT (39.04 mmol, 6.1 eq) and 14 mL of toluene were loaded into a flask under nitrogen.

A 2M solution of trimethyl aluminum in toluene (9.6 mL, 19.2 mmol, 3 eq) was added dropwise into the reaction mixture, while maintaining the temperature below 25 °C.

The system was stirred at 25 °C for 1 hour.

A solution of intermediate 9 (2 g, 6.4 mmol), obtained as described in the previous example, in methylene chloride (8 mL) was added to the reaction mixture cooled to -70 °C, while maintaining the temperature below -65 °C.

The system was stirred at -70 °C for 1 hour.

A 1.4 M solution of MeMgBr in toluene/THF 3/1 (13.7 mL, 19.2 mmol, 3 eq) was added to the reaction mixture, while maintaining the temperature below -65 °C.

The system was stirred at -70 °C for 1 hour.

The reaction was monitored by TLC analysis under the following conditions: TLC plate: silica gel on aluminium; starting substrate dissolved in dichloromethane; reaction mixture quenched in 20% citric acid solution in water and extracted with EtOAc; eluent: heptane/EtOAc 1/1; developer reagent: cerium phosphomolybdate.

Once the reaction was completed, a 20% citric acid solution (20 mL) was added to the system, while maintaining the temperature below -40 °C.

The system was stirred at 25 °C for 1 hour.

The phases were separated, and the aqueous phase was extracted with 16 mL of toluene.

The organic phase was washed with saturated sodium chloride solution (16 mL) and concentrated under reduced pressure at 25 °C to a waxy residue.

The residue was taken up with 32 mL of toluene and stirred at 25 °C until completely dissolved.
22 g of silica gel were added, and the system was stirred at 25 °C for 1 hour.

The reaction mixture was filtered, and the solid was washed with toluene (40 mL).

The silica gel (containing the product) was suspended in 40 mL of ethyl acetate.

The system was stirred at 25 °C for 1 hour.

The suspension was filtered, and the silica was washed with 20 mL of ethyl acetate.

The organic phase was concentrated under reduced pressure to a solid residue (2.2 g).

The residue was taken up with 19.5 mL of acetonitrile and stirred at 25 °C for 1 hour.

The suspension was filtered through a Büchner filter washing with 5 mL of acetonitrile.

The solid was dried under reduced pressure at 50 °C for 16 hours

1.8 g of white solid consisting of intermediate10 were obtained.

¹H-NMR (400 MHz, CDCl₃) δ 5.32-5.30 (m, 1H), 4.85 (s, 1H), 4.71 (s, 1H), 2.42 (dd, J=13.0, 1.9 Hz, 1H), 2.05-1.96 (m, 3H), 1.76 (s, 3H), 1.12 (s, 3H), 1.02 (s, 3H), 1.90-0.94 (m, 17H), 0.58 (s, 3H).

Mass spectrometry (ESI+): m/z = 311 [M+1-H₂O].

### EXAMPLE 4

This example refers to the implementation of step D) of the process of the invention.

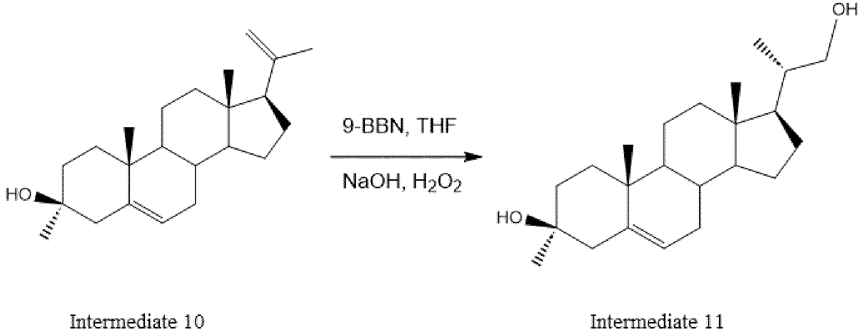

17.0 g of intermediate 10 (51.83 mmol), obtained as described in the previous example, and 14.4 g of 9-BBN dimer (59.60 mmol, 1.15 eq.) were loaded into a flask under nitrogen,

THF (114 mL), precooled to 10 °C, was added to the reaction mixture.

The system was heated to 60 °C and stirred for 1 hour.

30.2 mL of ethanol were added to the reaction mixture, cooled to 10 °C, and then 103.7 mL of 5 M sodium hydroxide solution (518.3 mmol, 10 eq.) were slowly added dropwise into the reaction mixture, while maintaining the temperature below 15 °C.

52.9 mL of 30% wt. hydrogen peroxide (518.3 mmol, 10 eq) were very slowly added dropwise to the reaction mixture, while maintaining the temperature below 15 °C.

The system was heated to 60 °C and stirred for 1 hour.

The reaction was monitored by TLC analysis under the following conditions: TLC plate: silica gel on aluminium; starting substrate dissolved in dichloromethane; reaction mixture extracted with EtOAc; eluent: heptane/EtOAc 1/1; developer reagent: cerium phosphomolybdate.

The reaction mixture, cooled to 25 °C, was filtered and the solid obtained was suspended in 60 mL of ethanol.

The system was heated to reflux for 1 hour and then to 25 °C for 1 hour.

The suspension was filtered and the solid obtained was suspended in 220 mL of water.

The system was heated to 80 °C and stirred for 1 hour.

The suspension, cooled to 25 °C, was filtered and the solid was dried under reduced pressure at 50 °C for 16 hours

12 g of a white solid consisting of intermediate 11 were obtained.

¹H-NMR (400 MHz, CDCl₃) δ 5.30-5.29 (m, 1H), 3.66-3.61 (m, 1H), 3.39-3.34 (m, 1H), 2.43-2.40 (m, 1H), 2.02-1.93 (m, 3H), 1.11 (s, 3H), 1.05 (d, J=6.6 Hz, 3H), 1.01 (s, 3H), 1.87-0.91 (m, 19H), 0.69 (s, 3H)

Mass spectrometry (ESI+): m/z = 329 [M+1-H₂O].

### EXAMPLE 5

This example refers to the implementation of step E) of the process of the invention.

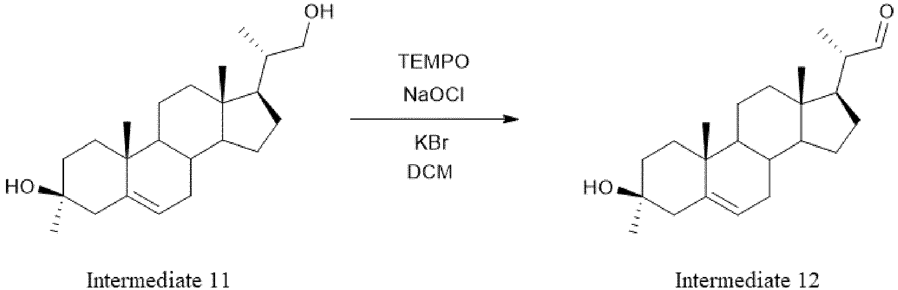

11.0 g of intermediate 11 (31.79 mmol), obtained as described in the previous example, and 132 mL of methylene chloride were loaded into a flask under nitrogen.

0.378 g of potassium bromide (3.18 mmol, 0.1 eq), 1.34 g of sodium bicarbonate (15.90 mmol, 0.5 eq) and 13.2 mL of water were loaded into another flask.

The aqueous solution obtained was added dropwise into the methylene mixture.

TEMPO (0.149 g, 0.95 mmol, 0.03 eq) and a sodium hypochlorite solution (13%, 22.8 mL, 44.51 mmol, 1.4 eq) were added to the reaction mixture, cooled to 0 °C, while maintaining the temperature below 5 °C.

The system was maintained at 0 °C and stirred for 2 hours.

The reaction was monitored by TLC analysis under the following conditions: TLC plate: silica gel on aluminum; starting substrate dissolved in dichloromethane; reaction mixture quenched in a 15% sodium thiosulfate solution in water and extracted with EtOAc; eluent: heptane/EtOAc 1/1; developer reagent: cerium phosphomolybdate.

A solution of sodium thiosulfate (3 g) in water (15 mL) was added to the reaction mixture, while maintaining the temperature below 5 °C.

The temperature was allowed to rise spontaneously up to 25 °C.

The phases were separated, and the aqueous phase was extracted with methylene chloride (150 mL).

The organic phase was washed with saturated sodium chloride solution (100 mL) and concentrated under reduced pressure to a small volume.

The residue was taken up with 150 mL of acetonitrile and then reconcentrated to a small volume.

The residue was taken up with 75 mL of acetonitrile, cooled to 0 °C and stirred for 1 hour.

The suspension was filtered through a Büchner filter, washing with cold acetonitrile.

The solid was dried under reduced pressure at 50 °C for 16 hours

6 g of white solid consisting of intermediate 12 were obtained.

¹H-NMR (400 MHz, CDCl₃) δ 9.57 (d, J=3.3 Hz, 1H), 5.31-5.29 (m, 1H), 3.66-3.61 (m, 1H), 2.44-2.32 (m, 1H), 2.02-1.94 (m, 3H), 1.13 (d, J=6.9 Hz, 3H), 1.11 (s, 3H), 1.02 (s, 3H), 1.92-0.93 (m, 17H), 0.73 (s, 3H)

Mass spectrometry (ESI+): m/z = 327 [M+1-H₂O].

### EXAMPLE 6

This example refers to the implementation of step F) of the process of the invention.

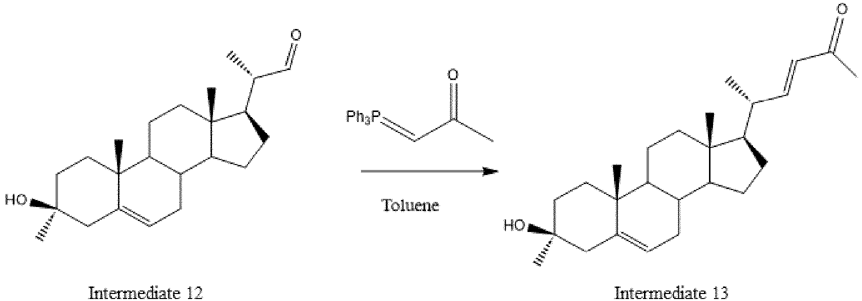

5 g of intermediate 12 (14.5 mmol), obtained as described in the previous example, and 13.6 g of 1-triphenylphosphoranylidene-2-propanone (43.5 mmol, 3 eq) were loaded into a flask under nitrogen.

100 mL of toluene were added to the mixture and the system was heated to 110 °C, under stirring, for 22 hours.

The reaction was monitored by UPLC analysis.

Once the reaction was completed, the solution was cooled to 25 °C, and concentrated under reduced pressure at 45 °C.

The crude product obtained was purified by column chromatography on silica gel eluting with a 90:10 mixture of toluene/isopropyl acetate (iPrOAc).

4 g of solid consisting of intermediate13 were obtained.

¹H-NMR (400 MHz, CDCl₃) δ 6.65 (dd, J=15.8, 8.9 Hz, 1H), 5.99 (d, J=16.0 Hz, 1H), 5.30-5.29 (m, 1H), 2.43-2.38 (m, 1H), 2.23 (s, 3H), 1.11 (s, 3H), 1.10 (d, J=5.0 Hz, 3H), 1.01 (s, 3H), 2.31-0.92 (m, 21H), 0.72 (s, 3H)

Mass spectrometry (ESI+): m/z = 385 [M+1] / 367 [M+1-H₂O].

### EXAMPLE 7

This example refers to the implementation of step G) of the process of the invention.

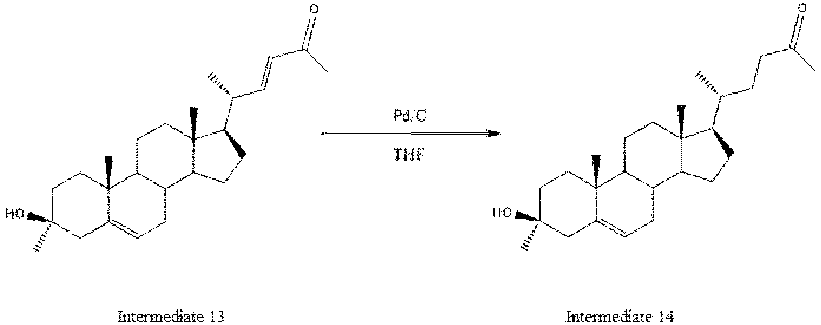

4 g of intermediate 13 (10.4 mmol), obtained as described in the previous example, and 120 mL of tetrahydrofuran were loaded into a flask under nitrogen.

0.4 g of palladium on carbon at 10% by weight (10% w/w) were added to the mixture.

The mixture was made inert with nitrogen, and then hydrogen was loaded at 0.2 bar.

The system was kept under stirring at 0.2 bar and at 25 °C for 16 hours.

The reaction was monitored by ¹H-NMR analysis in CDCl₃.

Once the reaction was completed, the mixture was filtered on Millipore, washed with THF and concentrated under reduced pressure at 45 °C.

4 g of solid consisting of intermediate 14 were obtained.

¹H-NMR (400 MHz, CDCl₃) δ 5.32-5.30 (m, 1H), 2.13 (s, 3H), 2.44-0.87 (m, 26H), 1.11 (s, 3H), 1.01 (s, 3H), 0.92 (d, J=6.4, 3H), 0.70 (s, 3H).

Mass spectrometry (ESI+): m/z = 369 [M+1-H₂O].

### EXAMPLE 8

This example refers to the implementation of step H) of the process of the invention.

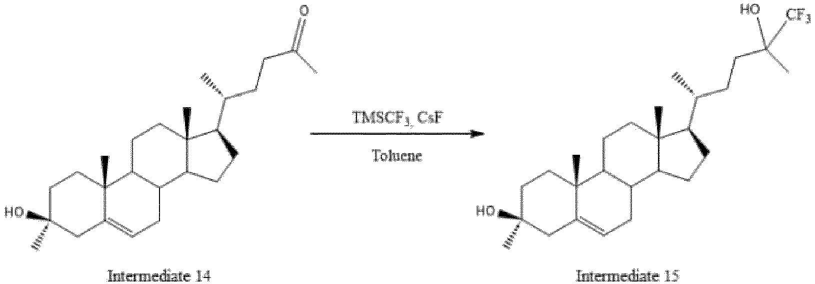

3.1 g of intermediate 14 (8.02 mmol), obtained as described in the previous example, and 23 mL of tetrahydrofuran were loaded into a flask under nitrogen.

2.44 g of cesium fluoride (16.05 mmol, 2 eq) were added to the reaction mixture.

The mixture was cooled to 10 °C and trimethyltrifluoromethyl silane (11.85 mL, 80.2 mmol, 10 eq) was slowly added to it, while maintaining the temperature below 10 °C.

The system was stirred at 25 °C for 16 hours.

The reaction was monitored by TLC analysis under the following conditions: TLC plate: silica gel on aluminium; starting substrate dissolved in dichloromethane; reaction mixture diluted in dichloromethane and deposited; eluent: toluene/iPrOAc 8/2; developer reagent: cerium phosphomolybdate.

Once the reaction was completed, a 1 M solution of tetra-n-butylammonium fluoride (TBAF) in THF (88.22 mL) was added to the reaction mixture.

The system was stirred at 35 °C for 4 hours.

The reaction was monitored by UPLC analysis.

Once the reaction was completed, 456 mL of methyl tert-butyl ether (MTBE) and 70 mL of saturated sodium bicarbonate solution were added to the system.

The system was stirred at 25 °C for 5 minutes.

The phases were separated, and the organic phase was washed twice with saturated sodium bicarbonate solution (2x70 mL) and then with 20 mL of water.

The organic phase was concentrated under reduced pressure at 45 °C.

The resulting crude (6.3 g) was purified by silica gel column chromatography eluting with a 95:5 mixture of toluene/iPrOAc.

2.2 g of solid consisting of the epimeric mixture 15 were obtained. The ratio of the 24S/24R diastereomers is 51:49, as determined by chiral HPLC analysis.

Mass spectrometry (ESI+): m/z = 439 [M+1-H₂O].

### EXAMPLE 9

This example refers to the implementation of steps I and J of the process of the invention, wherein Boc-Alanine (Boc-Ala) is used as the protected amino acid.

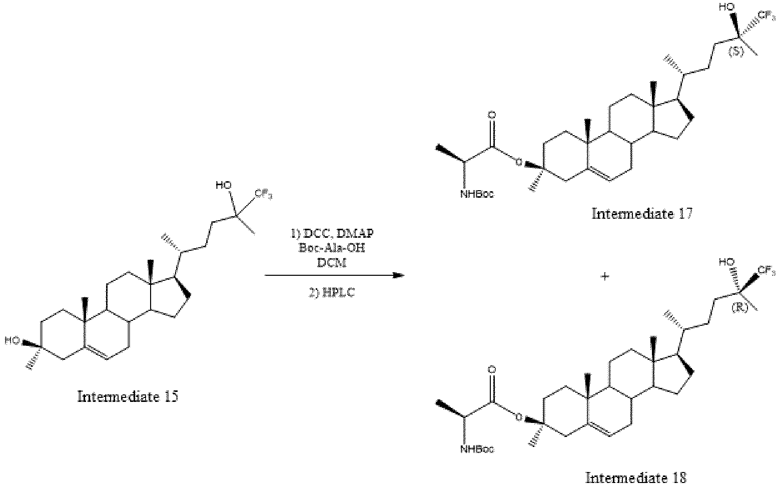

200 mg of mixture 15 (0.44 mmol), obtained as described in the previous example, and 10 mL of dichloromethane were loaded into a flask under nitrogen.

270 mg of DCC (1.31 mmol, 3 eq), 215 mg of DMAP (1.76 mmol, 2 eq) and 250 mg of Boc-Ala-OH (1.31 mmol, 3 eq) were added to the reaction mixture.

The system was stirred at 25 °C for 16 hours.

After 16 hours, a second addition of 270 mg of DCC (1.31 mmol, 3 eq), 215 mg of DMAP (1.76 mmol, 2 eq) and 250 mg of Boc-Ala-OH (1.31 mmol, 3 eq) was performed.

The system was stirred at 25 °C for 2 hours.

The reaction was monitored by TLC analysis under the following conditions: TLC plate: silica gel on aluminium; starting substrate dissolved in dichloromethane; reaction mixture quenched in water and extracted with EtOAc and deposited; eluent: toluene/iPrOAc 8/2; developer reagent: cerium phosphomolybdate.

Once the reaction was completed, 15 mL of water were added to the system, the organic phase was concentrated under reduced pressure at 45 °C, and the residue was taken up with 15 mL of toluene.

The phases were separated, and the organic phase was washed with 1 M hydrochloric acid solution (2x10 mL) and then with water (2x10 mL).

The organic phase was concentrated under reduced pressure at 45 °C to a solid residue, obtaining 318 mg of crude sample.

30 mg of crude sample were purified by preparative reversed-phase HPLC using the following conditions:
Eluent: water/acetonitrile 20:80 (isocratic method)
Column: Robusta^{®} C8 5u - 250 mm x 21.2 mm
Flow rate: 30 mL/min
UV detector wavelength: 200 nm
Sample: 30 mg dissolved in 1.5 mL of acetonitrile
Loop: 2 mL
From the chromatographic purification it was possible to isolate the diastereomer 17 (10 mg) and the diastereomer 18 (15 mg).
Intermediate 17 (24S)
¹H-NMR (400 MHz, DMSO-d6) δ 7.15 (d, J=7.2 Hz, 1H), 5.63 (s, 1H), 5.28 (brs, 1H), 3.84-3.79 (m, 1H), 2.29-2.25 (m, 1H), 1.92-0.81 (m, 24H), 1.34 (s, 9H), 1.26 (s, 3H), 1.16-1.13 (m, 6H), 0.93 (s, 3H), 0.83 (d, J=6.4 Hz, 3H), 0.60 (s, 3H)
Mass spectrometry (ESI+): m/z = 650 [M+1-Na].
Mass spectrometry (ESI-): m/z = 672 [M-1-HCOOH].
Intermediate 18 (24R)
¹H-NMR (400 MHz, DMSO-d6) δ 7.19 (d, J=7.6 Hz, 1H), 5.66 (s, 1H), 5.32 (brs, 1H), 3.82-3.79 (m, 1H), 2.33-2.30 (m, 1H), 1.97-0.85 (m, 24H), 1.38 (s, 9H), 1.31 (s, 3H), 1.23-1.19 (m, 6H), 0.97 (s, 3H), 0.89 (d, J=6.0 Hz, 3H), 0.65 (s, 3H)
Mass spectrometry (ESI+): m/z = 650 [M+1-Na].
Mass spectrometry (ESI-): m/z = 672 [M-1-HCOOH].

### EXAMPLE 10

This example refers to the implementation of step K of the process of the invention.

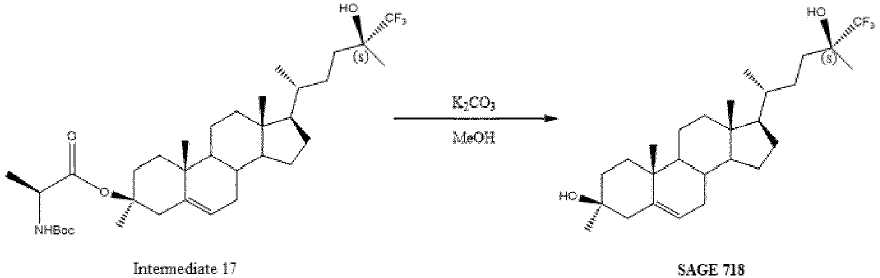

10 mg of intermediate 17 (0.016 mmol), obtained as described in the previous example, and 1 mL of methanol were loaded into a flask under nitrogen.

31 mg of potassium carbonate (0.22 mmol, 14 eq) were added to the reaction mixture, and the system was stirred at 65 °C for 16 hours.

The reaction was monitored by TLC analysis under the following conditions: TLC plate: silica gel on aluminium; starting substrate dissolved in dichloromethane; reaction mixture quenched in water and extracted with EtOAc and deposited; eluent: toluene/iPrOAc 8/2; developer reagent: cerium phosphomolybdate.

Once the reaction was completed, 2 mL of water were added to the system and the methanol was concentrated under reduced pressure at 45 °C.

The aqueous residue was neutralized by adding a 1 M hydrochloric acid solution and then extracted with 5 mL of EtOAc.

The phases were separated, and the aqueous phase was extracted with 5 mL of EtOAc.

The organic phase was washed with 5 mL of water and concentrated under reduced pressure at 45 °C to a solid residue.

10 mg of SAGE-718 (compound 5 of the article by Hill, J. Med. Chem. 2022, 65, 9063-9075) were obtained as a white solid whose ¹H-NMR, ¹³C-NMR and Ms analytical data coincide with those reported in the literature.

¹H-NMR (400 MHz, CDCl₃) δ 5.32-5.30 (m, 1H), 2.44-2.41 (m, 1H), 2.06-1.43 (m, 18H), 1.31 (d, J=6.4 Hz, 3H), 1.30-0.87 (m, 7H), 1.11 (s, 3H), 1.01 (s, 3H), 0.92 (m, 3H), 0.70 (s, 3H).

¹³C-NMR (400MHz, CDCl₃): 141.29; 128.11; 121.80; 73.65(q); 72.20; 56.85; 55.86; 50.60; 47.52; 42.47; 39.87; 37.11; 36.78; 36.70; 35.84; 32.05; 31.98; 31.66; 28.33; 28.15; 25.45; 24.30; 21.12; 20.02; 19.30; 18.64; 11.90.

Mass spectrometry (ESI+): m/z = 439 [M+1-H₂O].

Mass spectrometry (ESI-): m/z = 501 [M-1-HCOOH].

With a similar procedure, 10 mg of the 24R isomer (compound 16 of the article by Hill) were obtained, whose ¹H-NMR, ¹³C-NMR and Ms analytical data coincide with those reported in the literature.

¹H-NMR (400 MHz, CDCl₃) δ 5.32-5.30 (m, 1H), 2.44-2.41 (m, 1H), 2.06-1.43 (m, 18H), 1.31 (d, J=6.4 Hz, 3H), 1.30-0.87 (m, 7H), 1.11 (s, 3H), 1.01 (s, 3H), 0.92 (m, 3H), 0.70 (s, 3H).

¹³C-NMR (400MHz, CDCl₃): 141.20; 128.03; 121.80; 73.65(q); 72.24; 56.73; 55.50; 50.46; 47.42; 42.37; 39.75; 37.04; 36.68; 36.62; 35.62; 31.95; 31.95; 31.40; 28.23; 28.14; 25.41; 24.26; 21.05; 20.22; 19.27; 18.66; 11.85.

Mass spectrometry (ESI+): m/z = 439 [M+1-H₂O].

Mass spectrometry (ESI-): m/z = 501 [M-1-HCOOH].

### EXAMPLE 11

This example refers to an alternative method for separating SAGE-718 from the 24R diastereomer (steps I and J of the invention) via functionalization of the steroid with Boc-Valine (Boc-Val).

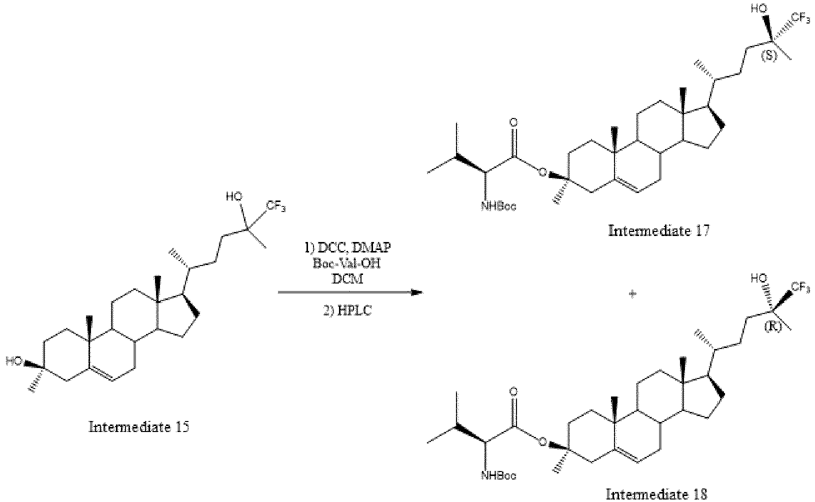

700 mg of epimeric mixture 15 (1.53 mmol), obtained as described in Example 8, and 28 mL of dichloromethane were loaded into a flask under nitrogen.

700 mg of DCC (3.37 mmol, 2.2 eq), 200 mg of DMAP (1.53 mmol, 1 eq), and 720 mg of Boc-Val-OH (3.37 mmol, 2.2 eq) were added to the reaction mixture.

The system was stirred at 25 °C for 16 hours.

The reaction was monitored by TLC analysis under the following conditions: TLC plate: silica gel on aluminium; starting substrate dissolved in dichloromethane; reaction mixture quenched in water and extracted with EtOAc and deposited; eluent: toluene/iPrOAc 8/2; developer reagent: cerium phosphomolybdate.

At the end of the reaction, 50 mL of water were added to the system, the organic phase was concentrated under reduced pressure at 45 °C and the residue was taken up with 50 mL of toluene.

The phases were separated, and the organic phase was washed with 1 M hydrochloric acid solution and then with water.

The organic phase was concentrated under reduced pressure at 45 °C to a solid residue, obtaining 1.5 g of crude sample.

200 mg of crude sample were purified by preparative reversed-phase HPLC using the following conditions:
Eluent: water/acetonitrile 15:85 (isocratic method)
Column: Robusta^{®} C8 5u - 250 mm x 21.2 mm
Flow rate: 30 mL/min
UV detector wavelength: 200 nm
Sample: 100 mg dissolved in 1.5 mL of acetonitrile
Loop: 2 mL
From the chromatographic purification it was possible to isolate the diastereomer 17 (90 mg) and the diastereomer 18 (80 mg).
Intermediate 17 (24S)
¹H-NMR (400 MHz, DMSO-d6) δ 7.03 (d, J=8.0 Hz, 1H), 5.67 (s, 1H), 5.33 (brs, 1H), 3.70-3.67 (m, 1H), 2.58-2.54 (m, 1H), 2.34-2.32 (m, 1H), 2.00-0.91 (m, 24H), 1.39 (s, 9H), 1.28 (s, 3H), 1.17 (s, 3H), 0.97 (s, 3H), 0.90-0.86 (m, 9H), 0.65 (s, 3H)
Mass spectrometry (ESI+): m/z = 678 [M+1-Na].
Intermediate 18 (24R)
¹H-NMR (400 MHz, DMSO-d6) δ 7.03 (d, J=8.0 Hz, 1H), 5.66 (s, 1H), 5.33 (brs, 1H), 3.70-3.67 (m, 1H), 2.58-2.55 (m, 1H), 2.34-2.31 (m, 1H), 2.00-0.91 (m, 24H), 1.39 (s, 9H), 1.32 (s, 3H), 1.19 (s, 3H), 0.97 (s, 3H), 0.90-0.86 (m, 9H), 0.65 (s, 3H)
Mass spectrometry (ESI+): m/z = 678 [M+1-Na].

### EXAMPLE 12

This example refers to an alternative method for separating SAGE-718 from the 24R diastereomer (steps I and J of the invention) via functionalization of the steroid with Boc-Glycine (Boc-Gly).

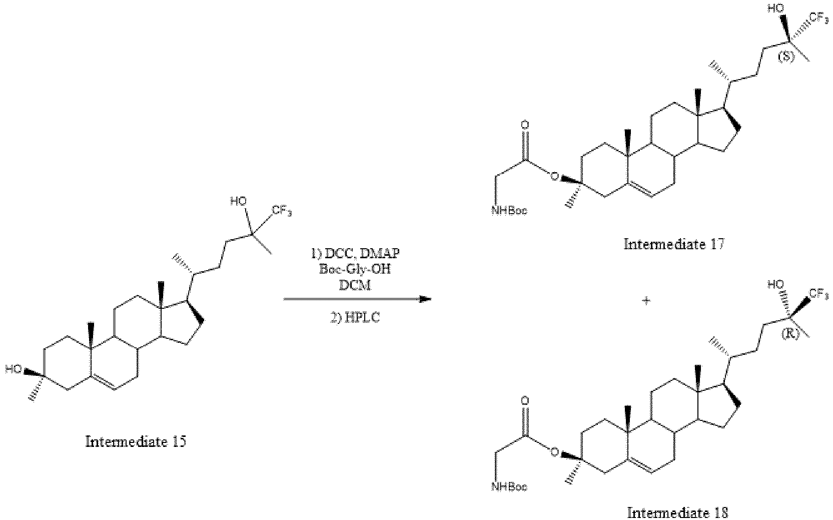

20 mg of epimeric mixture 15 (0.04 mmol), obtained as described in Example 8, and 2 mL of dichloromethane were loaded into a flask under nitrogen.

27 mg of DCC (0.13 mmol, 3 eq), 11 mg of DMAP (0.09 mmol, 2 eq), and 230 mg of Boc-Gly-OH (0.13 mmol, 3 eq) were added to the reaction mixture.

The system was stirred at 25 °C for 16 hours.

After 16 hours, a second addition of 27 mg of DCC (0.13 mmol, 3 eq), 11 mg of DMAP (0.09 mmol, 2 eq) and 230 mg of Boc-Gly-OH (0.13 mmol, 3 eq) was performed.

The system was stirred at 25 °C for 2 hours

The reaction was monitored by TLC analysis under the following conditions: TLC plate: silica gel on aluminium; starting substrate dissolved in dichloromethane; reaction mixture quenched in water and extracted with EtOAc and deposited; eluent: toluene/iPrOAc 8/2; developer reagent: cerium phosphomolybdate.

Once the reaction was completed, 5 mL of water were added to the system, the organic phase was concentrated under reduced pressure at 45 °C and the residue was taken up with 5 mL of toluene.

The phases were separated, and the organic phase was washed with 1 M hydrochloric acid solution and then with water.

The organic phase was concentrated under reduced pressure at 45 °C to a solid residue thus obtaining 30 mg of crude sample.

From the analytical HPLC analysis carried out on the crude sample obtained, it was possible to observe the chromatographic separation between the two diastereomers 17 and 18.

## Claims

1. Process for the synthesis of a mixture of epimers (3β,24*S*)-25,25,25-trifluoro-3-methyl-26,27-dinorergost-5-ene-3,24-diol and (3(3,24*R*)-25,25,25-trifluoro-3-methyl-26,27-dinorergost-5-ene-3,24-diol, comprising the following steps:
A) introduction of a methylene group in position 20 of the compound 5-pregnenolone via Wittig reaction, obtaining intermediate 8: 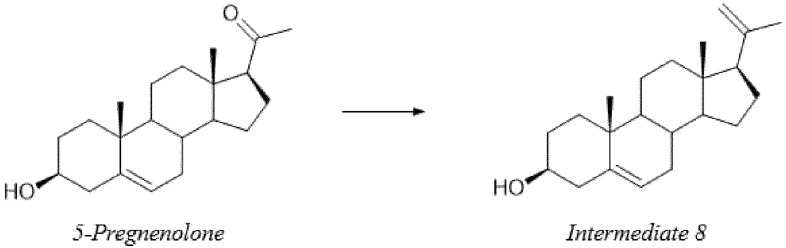
B) oxidation to carbonyl of the hydroxyl in position 3 of intermediate 8 obtaining intermediate 9: 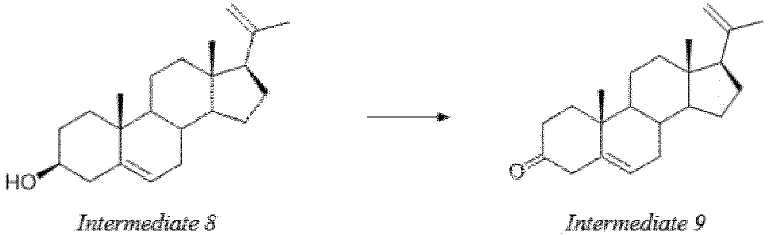
C) introduction of a methyl group in position 3 of intermediate 9 via Grignard reaction obtaining intermediate 10: 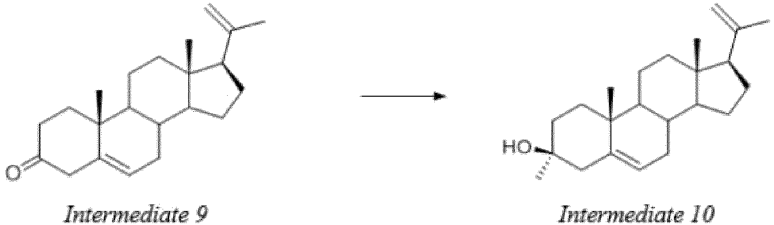
D) hydroboration-oxidation of the methylene group in position 21 of intermediate 10 obtaining intermediate 11: 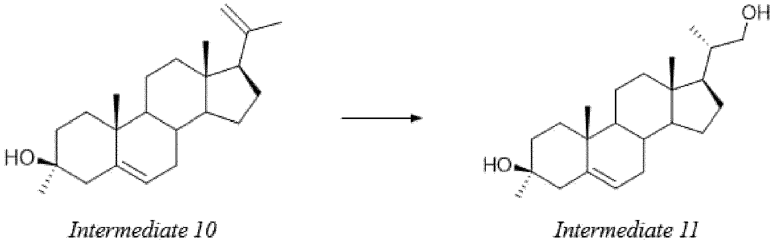
E) oxidation to carbonyl of the hydroxyl in position 22 of intermediate 11 obtaining intermediate 12: 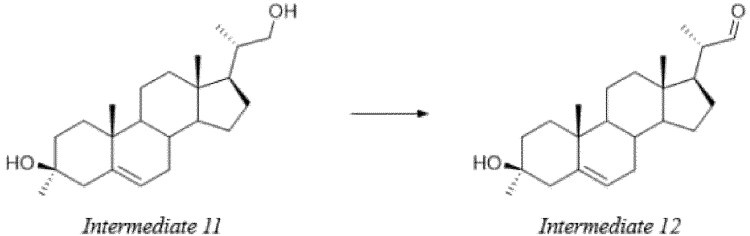
F) introduction of a 2-propanone radical in position 22 of intermediate 12 via Wittig reaction obtaining intermediate 13: 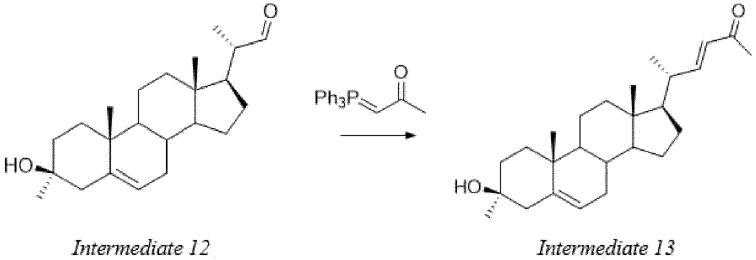
G) reduction of the double bond in position 22 of intermediate 13 obtaining intermediate 14: 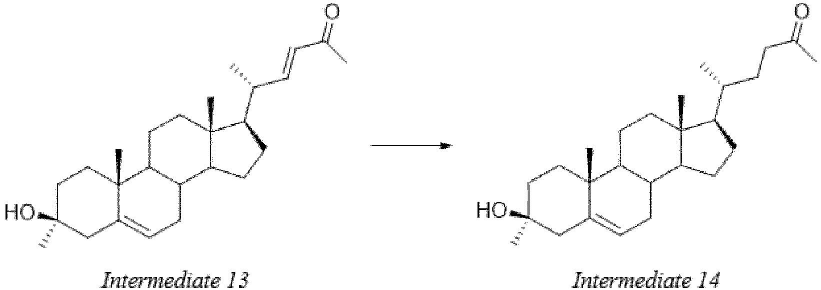
H) introduction of a trifluoromethyl radical in position 24 of intermediate 14 obtaining the epimeric mixture 15:

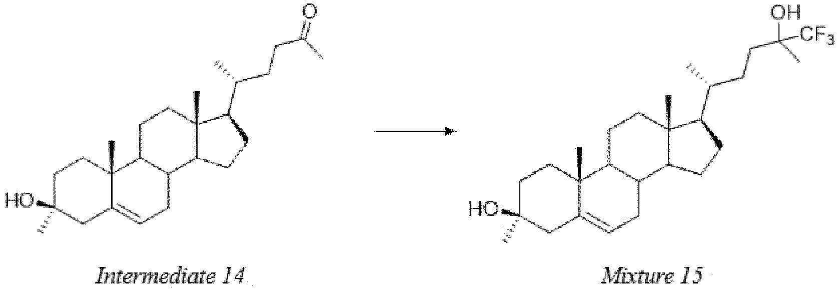

2. Process according to claim 1, wherein step A) is carried out at a temperature between 15 and 60 °C for a time between 1.5 and 6 hours, generating a ylide *in situ* starting from potassium tert-butylate and a methyltriphenylphosphonium salt selected from methyltriphenylphosphonium bromide, methyltriphenylphosphonium chloride, methyltriphenylphosphonium iodide and methyltriphenylphosphonium methylcarbonate, and using potassium tert-butylate in a molar ratio between 1.5 and 2.5 with respect to 5-pregnenolone, and the methyltriphenylphosphonium salt in a molar ratio between 1.5 and 2.5 with respect to 5-pregnenolone.

3. Process according to any one of claims 1 or 2, wherein the oxidation reaction of step B) is carried out using an oxidizing system selected among: chromium (VI) compounds; a hypochlorite of an alkaline or alkaline earth metal in the presence of 2,2,6,6-tetramethylpiperidin-1-oxyl radical or a derivative thereof selected from 4-hydroxy-2,2,6,6-tetramethylpiperidin-1-oxyl radical, 4-methoxy-2,2,6,6-tetramethylpiperidin-1-oxyl radical and 4-benzyloxy-2,2,6,6-tetramethylpiperidin-1-oxyl radical; a ruthenium derivative selected from ruthenium tetroxide and ruthenium trichloride in the presence of a reoxidant selected from sodium hypochlorite and tetrapropylammonium perruthenate in the presence of N-methylmorpholine N-oxide; potassium peroxymonosulfate KHSO₅; 1,3,5-trichloro-2,4,6-triazinetrione; hypervalent iodine compounds; and a system comprising oxalyl chloride, triethylamine and dimethyl sulfoxide.

4. Process according to claim 3, wherein said oxidizing system is the system comprising oxalyl chloride, triethylamine and dimethyl sulfoxide respectively used in a molar ratio between 1.5 and 2, between 6 and 7, and between 3 and 4 with respect to intermediate 8; the reaction is carried out in dichloromethane at a temperature between -75 and 25 °C for a time between 1.5 and 6 hours, and at the end of the reaction intermediate 9 is recovered by distillation of dichloromethane at a pressure lower than 50 mbar and a temperature not exceeding 25 °C.

5. Process according to any one of the preceding claims, wherein the reaction of step C is carried out with a Grignard reagent of formula CH₃MgX, wherein X stands for an halogen atom selected among chlorine and bromine, in the presence of trimethyl aluminum and the compound 2,6-bis(1,1-dimethylethyl)-4-methylphenol, and wherein the Grignard reagent, trimethyl aluminum and 2,6-bis(1,1-dimethylethyl)-4-methylphenol are respectively used in a molar ratio between 3 and 4, between 3 and 4, and between 6.1 and 8.1 with respect to intermediate 9.

6. Process according to claim 5, wherein methylmagnesium bromide is used as a Grignard reagent, dichloromethane is used as a solvent, and the reaction is carried out at a temperature between -75 and 25 °C for a time between 3 and 6 hours.

7. Process according to any one of the preceding claims, wherein the hydroboration reaction of step D is carried out with a boron compound or a complex containing a boron compound, said compound or complex being selected from the borane-tetrahydrofuran complex, the borane-diethyl ether complex, the borane-dimethylsulfide complex, the borane-pyridine complex, bis(1,2-dimethylpropyl)borane, texylborane, 9-borabicyclo[3.3.1]nonane, and 9-borabicyclo[3.3.1]nonane dimer, operating at a temperature between 5 and 65 °C for a time between 2 and 6 hours.

8. Process according to any one of the preceding claims, wherein the oxidation reaction of step D is carried out using hydrogen peroxide in alkaline conditions.

9. Process according to claim 8, wherein 30% by weight hydrogen peroxide and a 5M sodium hydroxide solution are used, and wherein 9-borabicyclo[3.3.1]nonane dimer, hydrogen peroxide, and sodium hydroxide are respectively used in a molar ratio between 1.15 and 3, between 10 and 15, and between 10 and 15 with respect to intermediate 10.

10. Process according to any one of the preceding claims, wherein the oxidation reaction of step E is carried out using an oxidizing system selected among: chromium (VI) compounds; a hypochlorite of an alkaline or alkaline earth metal in the presence of 2,2,6,6-tetramethylpiperidin-1-oxyl radical or a derivative thereof selected from 4-hydroxy-2,2,6,6-tetramethylpiperidin-1-oxyl radical, 4-methoxy-2,2,6,6-tetramethylpiperidin-1-oxyl radical, and 4-benzyloxy-2,2,6,6-tetramethylpiperidin-1-oxyl radical; a ruthenium derivative selected from ruthenium tetroxide and ruthenium trichloride in the presence of a reoxidant selected from sodium hypochlorite and tetrapropylammonium perruthenate in the presence of N-methylmorpholine N-oxide; potassium peroxymonosulfate KHSOs; 1,3,5-trichloro-2,4,6-triazinetrione; hypervalent iodine compounds; and a system comprising oxalyl chloride, triethylamine and dimethyl sulfoxide.

11. Process according to claim 10, wherein said oxidation reaction is carried out with calcium or sodium hypochlorite in the presence of 2,2,6,6-tetramethylpiperidin-1-oxyl radical, sodium bicarbonate, and a salt selected from potassium bromide and sodium bromide, operating in a solvent selected among dichloroethane, ethyl acetate, acetonitrile and dichloromethane, at a temperature between 0 and 5 °C for a time between 2 and 4 hours, and wherein the calcium or sodium hypochlorite, sodium bicarbonate, potassium or sodium bromide, and 2,2,6,6-tetramethylpiperidin-1-oxyl radical are respectively used in a molar ratio between 1.4 and 2.0, between 0.5 and 1.5, between 0.1 and 0.5, and between 0.03 and 0.06 with respect to intermediate 11.

12. Process according to any one of the preceding claims, wherein the reaction of step F is carried out using 1-triphenylphosphoranylidene-2-propanone in a molar ratio between 2 and 4 with respect to intermediate 12, operating at a temperature between 25 and 115 °C for a time between 12 and 24 hours.

13. Process according to any one of the preceding claims, wherein the reaction of step G is carried out by catalytic hydrogenation, using palladium on carbon at 5% or 10% by weight as a catalyst, and operating with a hydrogen pressure between 0.1 and 1.0 bar in a solvent selected among methanol, ethyl acetate and tetrahydrofuran, at a temperature between 15 and 30 °C, and for a time of at least 16 hours.

14. Compound (3β)-3-hydroxy-3-methyl-26,27-dinorcholest-5,22-dien-24-one, having the following structural formula:

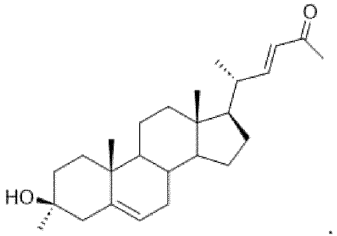

15. Process for the separation of a mixture of epimers (3β,24*S*)-25,25,25-trifluoro-3-methyl-26,27-dinorergost-5-ene-3,24-diol and (3β,24*R*)-25,25,25-trifluoro-3-methyl-26,27-dinorergost-5-ene-3,24-diol, mixture 15, comprising the following steps:
I) esterification of the hydroxyl in position 3 of said epimeric mixture 15 with a protected aliphatic amino acid obtaining intermediate 16, in turn in the form of an epimeric mixture: 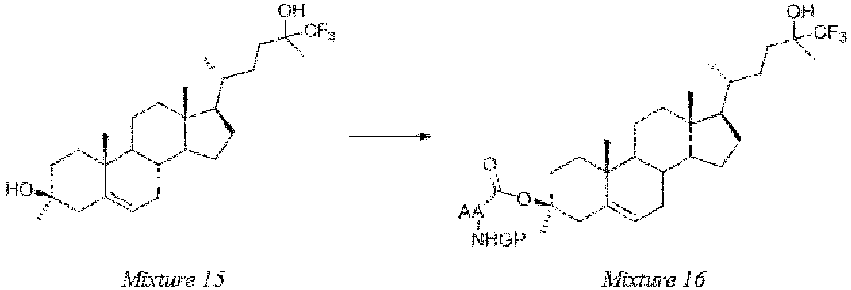 where, in the formula of mixture 16, AA is the radical of the amino acid and GP is the protective group linked to the amino function of the amino acid;
J) chromatographic separation of the epimeric mixture 16 obtaining the two epimers 17 and 18, where the epimer 17 has the desired configuration: 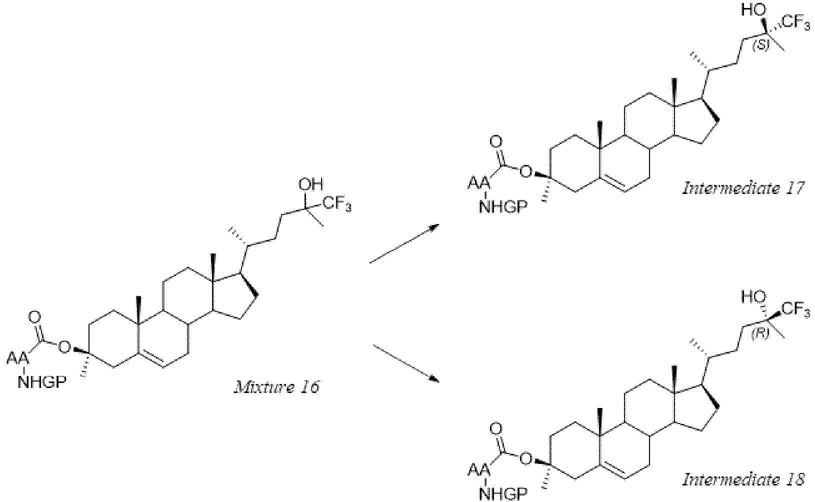
K) removal by hydrolysis of the ester in position 3 of intermediate 17, obtaining the product (3β,24*S*)-25,25,25-trifluoro-3-methyl-26,27-dinorergost-5-ene-3,24-diol (SAGE-718) as a pure epimer:
